# EUROPÄISCHE PATENTSCHRIFT

(11) **EP 3 184 096 B1**
(45) Veröffentlichungstag und Bekanntmachung des Hinweises auf die Patenterteilung: **07.11.2018**
(21) Anmeldenummer: 15201791.9
(22) Anmeldetag: 21.12.2015
(51) Int. Cl.: A61K 6/00, A61K 6/083

(54) **DENTALMATERIALIEN MIT LICHTINDUZIERTER REVERSIBLER FARBGEBUNG**
DENTAL MATERIALS WITH LIGHT-INDUCED REVERSIBLE COLOURING
MATERIAUX DENTAIRES A COLORATION REVERSIBLE INDUITE PAR LA LUMIERE

(43) Veröffentlichungstag der Anmeldung: 28.06.2017
(73) Patentinhaber: Ivoclar Vivadent AG, 9494 Schaan (LI)
(72) Erfinder: MOSZNER, Norbert, 9495 Triesen (LI); BOCK, Thorsten, 6800 Feldkirch (AT); HAUNER, Martina, 6800 Feldkirch (AT); GIANASMIDIS, Alexandros, 9436 Balgach (CH); LAMPARTH, Iris, 9472 Grabs (CH); STREHMEL, Bernd, 47798 Krefeld (DE); BRÖMME, Thomas, 47798 Krefeld (DE)
(74) Vertreter: Uexküll & Stolberg

(56) Entgegenhaltungen:
- EP-A2- 0 744 172
- US-A1- 2011 216 273
- IRIE M: "Diarylethens for Memories and Switches", CHEMICAL REVIEWS, AMERICAN CHEMICAL SOCIETY, WASHINGTON, DC, US, Bd. 100, 1. Januar 2000 (2000-01-01), Seiten 1685-1716, XP002267352, DOI: 10.1021/CR980069D
- BOIKO Y: "Improvement of thermal stability in photochromic holograms", OPTICS LETTERS, OPTICAL SOCIETY OF AMERICA, US, Bd. 34, Nr. 8, 15. April 2009 (2009-04-15) , Seiten 1279-1281, XP001522825, ISSN: 0146-9592, DOI: 10.1364/OL.34.001279

## Beschreibung

Die vorliegende Erfindung betrifft thermisch- und/oder lichthärtende Zusammensetzungen mit lichtinduzierter reversibler Farbgebung, wie Polymerisationsharze und Komposite, die sich insbesondere als dentale Füllungskomposite, Verblendmaterialien, dentale Adhäsive und dentale Beschichtungsmaterialien sowie zur Herstellung von Inlays und Onlays eignen.

Bei Kunststoffen werden dem Polymer Farbmittel, man unterscheidet zwischen Farbstoffen und Pigmenten, zur Einstellung der gewünschten Farbe und Transparenz als Additive zugesetzt. Farbstoffe sind organischer Natur und meist in organischen Lösungsmitteln löslich, während Pigmente feste Teilchen mit Korngrößen zwischen ca. 0,01 bis ca. 1 µm sind und in organische und anorganische Pigmente unterteilt werden (vgl. Taschenbuch der Kunststoff-Additive, Hrsg. R. Gächter, H. Müller, 3. Aufl., Carl Hanser Verlag, München und Wien 1989, 663-736). Bei zahnärztlichen Dentalmaterialien und insbesondere bei hochästhetischen Füllungskompositen werden zur Einstellung der Farbe Mischungen verschiedener anorganischer Pigmente eingesetzt, die sich durch geringe Löslichkeit in organischen Lösungsmitteln und Fetten sowie durch eine sehr gute Farbstabilität auszeichnen. Neben der permanenten Färbung von Dentalmaterialien ist es in manchen Situationen vorteilhaft, das Dentalmaterial temporär durch reversible Farbgebung besser sichtbar machen zu können. Beispiele hierfür sind eine reversible Farbgebung zur Erkennung dünner Schichten, wie im Falle von mit Adhäsiv behandelten Zahnoberflächen oder Zementüberschüssen, sowie die Sichtbarmachung von Fissurenversieglern.

Die EP 0 744 172 A1 offenbart photochrome Dentalwerkstoffe, die ein photochromes Material wie beispielsweise einen photochromen Farbstoff, ein photochromes Glas, eine photochrome Keramik und/oder eine photochrome Glaskeramik enthalten. Der photochrome Dentalwerkstoff lässt sich durch kurze Bestrahlung mit Licht in einen gefärbten Zustand überführen, der eine bessere Unterscheidung von der natürlichen Zahnhartsubstanz möglich macht. Nachteilig ist, dass sich die anschließende Entfärbung zum Teil über mehrere Stunden hinzieht, was die lichtinduzierte Materialaushärtung beeinträchtigen kann. Außerdem kann der Zahnarzt die endgültige Farbgebung der Restauration nicht in einer Sitzung kontrollieren.

Man unterscheidet photochrome Stoffe vom T-Typ, bei der die Rückreaktion nach Beendung der Bestrahlung vorwiegend thermisch abläuft, und Stoffe vom P-Typ, bei denen die Rückreaktion vorwiegend photochemisch abläuft, d.h. durch Licht einer anderen Wellenlänge ausgelöst wird (vgl. S. N. Corns, A. M. Partingtom, A. D. Towns, Color. Technol. 125 (2009) 249-261). Beispiele für photochrome Stoffe des P-Typs sind Diarylethene mit heterocyclischen Arylgruppen (vgl. M. Irie, Chem. Rev. 100 (2000) 1685-1716). Die Verwendung von photochromen Diarylethenen in Dentalmaterialien ist nach dem Stand der Technik nicht bekannt.

Der Erfindung liegt die Aufgabe zugrunde, Werkstoffe für dentale Anwendungen bereitzustellen, deren Farbe sich durch Bestrahlen mit Licht reversibel ändern lässt und die nicht die mit dem Stand der Technik verbundenen Nachteile aufweisen.

Die Aufgabe wird erfindungsgemäß durch radikalisch polymerisierbare Dentalwerkstoffe, gelöst, die mindestens eine Verbindung gemäß der allgemeinen Formel I, mindestens ein radikalisch polymerisierbares Monomer und mindestens einen Initiator für die radikalische Polymerisation enthalten:

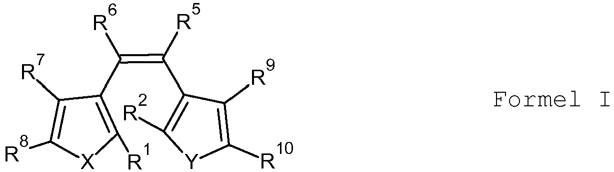

Die Variablen der Formel I haben die folgenden Bedeutungen:
- X, Y: jeweils S;
- R¹,R²: jeweils Methyl;
- R⁵;R⁶: bilden zusammen eine -(CH₂)ₙ- Gruppe, wobei n 3 ist und wobei in der -(CH₂)- Gruppe ein oder mehrere H-Atome, vorzugsweise alle H-Atome, durch F ersetzt sein können;
- R⁷,R⁹: unabhängig voneinander jeweils H oder ein C₁-C₃-Alkylrest;
- R⁸,R¹⁰: unabhängig voneinander jeweils ein C₁-C₆-Alkylrest, der durch O oder -O-C(=O)-NH- unterbrochen sein kann und der eine endständige radikalisch polymerisierbare Gruppe trägt, vorzugsweise eine Methacrylatgruppe.

Durch die Formel (I) und die übrigen hierin gezeigten Formeln werden sämtliche stereoisomere Formen sowie Gemische verschiedener stereoisomerer Formen, wie z.B. Racemate, erfasst. Die Formeln erfassen nur solche Verbindungen, die mit der chemischen Valenzlehre vereinbar sind. Der Hinweis, dass ein Rest durch ein Heteroatom wie O unterbrochen sein kann, ist so zu verstehen, dass die O-Atome in die Kohlenstoffkette oder den Kohlenstoffring des Restes eingeschoben werden, d.h. beidseitig von Kohlenstoffatomen begrenzt werden. Die Anzahl der Heteroatome ist daher um mindestens 1 kleiner als die Zahl der Kohlenstoffatome, und die Heteroatome können nicht endständig sein. Bei Kohlenwasserstoffresten, die Kohlenstoff- und Heteroatome enthalten, ist die Anzahl der Heteroatome ohne Berücksichtigung von Substituenten stets kleiner als die Zahl der Kohlenstoffatome. C₁-Reste können nicht unterbrochen sein.

Bevorzugte polymerisationsfähige Gruppen, die bei den Resten R als Substituenten vorhanden sein können, sind Vinyl, Styryl, Acrylat (CH₂=CH-CO-O-), Methacrylat (CH₂=C(CH₃)-CO-O-), Acrylamid (CH₂=CH-CO-NR¹⁴- mit R¹⁴ = H oder C₁-C₈-Alkyl), Methacrylamid (CH₂=C(CH₃)-CO-NH-), besonders bevorzugt (Meth)acrylat, Methacrylamid und/oder N-Alkylacrylamid.

Bevorzugte Verbindungen der Formel I sind:

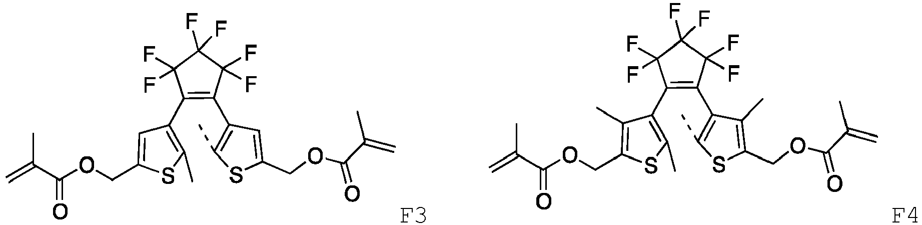

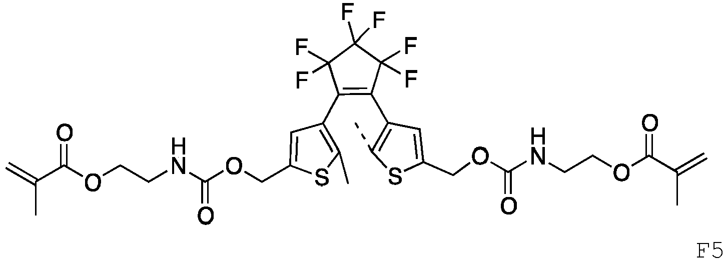

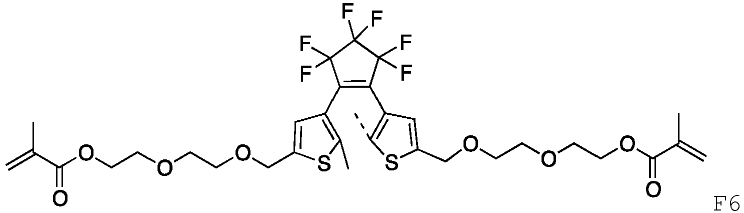

Die Verbindungen der Formel I tragen zwei radikalisch polymerisierbare Gruppen, insbesondere Vinyl-, (Meth)acryl- und/oder (Meth)acrylamidgruppen. Solche Verbindungen werden bei der radikalischen Aushärtung der Dentalmaterialien in die organische Polymermatrix kovalent eingebaut und sind danach nicht mehr auswaschbar, so dass die photochromen Eigenschaften auch über längere Zeiträume erhalten bleiben. Weiterhin wird auf diese Weise die Migrationsfähigkeit deutlich herabgesetzt.

Die erfindungsgemäß verwendeten Verbindungen der Formel I zeichnen sich durch eine reversible lichtinduzierte Farbgebung bei unterschiedlicher Wellenlänge aus, d.h., sie lassen sich durch kurzzeitiges Bestrahlen mit Licht der Wellenlänge λ₁ Färben und durch Bestrahlen mit Licht der Wellenlänge λ₂ wieder Entfärben, wobei die Entfärbung vorzugsweise mit langwelligerem sichtbarem Licht erfolgt. Dieser Vorgang läuft überraschenderweise mit hoher Effizienz ab, selbst dann, wenn die Verbindungen der Formel I durch Copolymerisation in amorphe Polymernetzwerke, sog. Polymergläser, eingebunden sind. Erfindungsgemäß sind Verbindungen der Formel I bevorzugt, bei denen λ₂ > λ₁ + 50 nm ist. λ₁ liegt dabei vorzugsweise im ultravioletten Bereich und λ₂ im sichtbaren Spektralbereich. Dabei soll λ₁ so gewählt werden, dass bei Bestrahlung ein enthaltener Photoinitiator und die Verbindung I sich nicht wesentlich in der Funktionalität beeinflussen. Die Einarbeitung von I in den Dentallack führt zu keiner sichtbaren Verfärbung der Versiegelung.

Die Verbindungen der Formel I sind P-Typ Chromophore. Dentalwerkstoffe, die eine Verbindung der Formel I enthalten, können durch Bestrahlen mit Licht der Wellenlänge λ2 gezielt entfärbt werden. Dies ermöglicht eine große Flexibilität bei der Bearbeitung der Werkstoffe, weil die Bearbeitungszeit anders als bei T-Typ Chromophoren beliebig gewählt werden kann.

Die erfindungsgemäßen Dentalmaterialien auf der Basis der photochrome Additive der allgemeinen Formel I ermöglichen somit eine gezielte Farbgebung bzw. Sichtbarmachung durch kurzzeitiges Bestrahlen mit Licht der Wellenlänge λ₁. Durch kurzzeitiges Bestrahlen im Sekundenbereich mit Licht der Wellenlänge λ₂ lassen sich die verfärbten Dentalmaterialien anschließend wieder entfärben. Die dabei ablaufenden Reaktionen sind im Folgenden beispielhaft gezeigt:

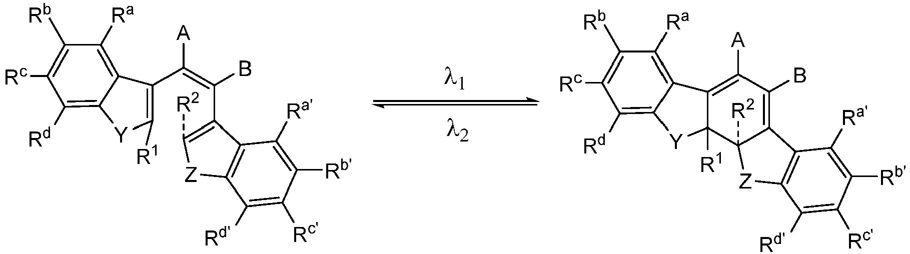

Die ungefärbten Verbindungen zeichnen sich durch einen offenen Ring (linke Formel), die gefärbten Verbindungen durch einen geschlossenen Ring aus (rechte Formel). Die erfindungsgemäßen Werkstoffe werden vorzugsweise in der ungefärbten Form vertrieben, und dementsprechend werden die Verbindungen der Formel I hier durch Verwendung der Formeln für die ungefärbten Verbindungen definiert. Es versteht sich jedoch von selbst, dass auch solche Werkstoffe, die die entsprechenden gefärbten Verbindungen enthalten, Gegenstand der Erfindung sind.

Dabei hängt der erzielbare Farbeffekt vor allem von der Konzentration an photochromer Verbindung der Formel I, dem Umwandlungsgrad, der Umwandlungsgeschwindigkeit und dem Extinktionskoeffizient der photochemisch erzeugten Verbindung ab. Bevorzugt sind Verbindungen, welche eine Quantenausbeute von mehr als 10% besitzen. Der Extinktionskoeffizient liegt vorzugsweise über 10000 M⁻¹ cm⁻¹.

Erfindungsgemäß sind solche Verbindungen der Formel I bevorzugt, die mit einer Wellenlänge λ₁ kleiner als 400 nm, insbesondere mit UV-Licht im Bereich von 320-395 nm angeregt werden können, wobei solche Verbindungen besonders bevorzugt sind, die keine oder nur wenig Eigenfarbe aufweisen. Die Formeln F3-F6 entsprechen diesen Ansprüchen.

Die erfindungsgemäßen Dentalmaterialien enthalten neben den Monomeren der allgemeinen Formel I zusätzlich weitere radikalisch polymerisierbare Monomere, insbesondere mono- und/oder polyfunktionelle (Meth)acrylsäurederivate. Besonders bevorzugt sind Werkstoffe, die als radikalisch polymerisierbares Monomer mindestens ein multifunktionelles (Meth)acrylat oder eine Mischung von mono- und multifunktionellen (Meth)acrylaten enthalten. Unter monofunktionellen (Meth)acrylaten werden Verbindungen mit einer, unter polyfunktionellen (Meth)acrylaten Verbindungen mit zwei oder mehr, vorzugsweise 2 bis 4 radikalisch polymerisierbaren Gruppen verstanden. Gemäß einer ganz besonders bevorzugten Ausführungsform enthalten die erfindungsgemäßen Zusammensetzungen mindestens ein Dimethacrylat oder eine Mischung von Mono- und Dimethacrylaten. Materialien, die als radikalisch polymerisierbares Monomer mono- und multifunktionelle (Meth)acrylaten enthalten, eignen sich besonders als Dentalwerkstoffe. In allen Fällen sind Methacrylate als CoMonomere bevorzugt. Es wurde gefunden, dass die Monomere der Formel I und insbesondere die bevorzugten Verbindungen der Formel I mit den hier genannten Co-Monomeren gut verträglich sind und homogene Mischungen bilden, die bei der Polymerisation Materialien mit sehr guten mechanischen Eigenschaften ergeben.

Bevorzugte mono- oder polyfunktionelle Methacrylate sind Methyl-, Ethyl-, Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat (HEMA) und 2,2,4-Trimethylhexamethylendiisocyanat), TMX-UDMA (ein Additionsprodukt aus einer Mischung von HEMA und Hydroxypropylmethacrylat (HPMA) mit α,α,α',α'-Tetramethyl-m-xylylendiisocyanat (TMXDI)), Bis(methacryloyloxymethyl)tricyclo[5.2.1.]decan (TCDMA), ethoxy- oder propoxyliertes Bisphenol-A-di(meth)acrylat, wie z.B. das Bisphenol-A-dimethacrylat 2-[4-(3-Methacryloyloxyethoxyethyl)-phenyl]-2-[4-(3-methacryloyloxyethyl)phenyl]-propan) (SR-348c) mit 3 Ethoxygruppen oder 2,2-Bis[4-(2-(meth)acryloxypropoxy)-phenyl]propan, Di-, Tri- oder Tetraethylenglycoldi(meth)-acrylat, Trimethylolpropantri(meth)acrylat, Pentaerythrit-tetra(meth)acrylat, sowie Glycerindi(meth)acrylat, 1,4-Butandioldi(meth)acrylat, 1,10-Decandioldi(meth)acrylat oder 1,12-Dodecandioldi(meth)acrylat oder Glycerintrimethacrylat (GTMA).

Weiter bevorzugt sind N-mono- oder -disubstituierte Acrylamide, wie z.B. N-Ethylacrylamid, N,N-Dimethylacrylamid, N-(2-Hydroxyethyl)acrylamid oder N-Methyl-N-(2-hydroxyethyl)acrylamid, bzw. N-monosubstituierte Methacrylamide, wie z.B. N-Ethylmethacrylamid oder N-(2-Hydroxyethyl)ethacrylamid sowie N-Vinylpyrrolidon. Diese Monomere zeichnen sich durch eine geringe Viskosität und eine hohe Hydrolysestabilität aus und eignen sich besonders als Verdünnermonomere.

Ebenfalls bevorzugt sind vernetzende Pyrrolidone, wie z.B. 1,6-Bis(3-vinyl-2-pyrrolidonyl)-hexan oder kommerziell zugängliche Bisacrylamide, wie Methylen- oder Ethylenbisacrylamid, bzw. Bis(meth)acrylamide, wie z.B. N,N'-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsäurechlorid synthetisiert werden können. Diese Monomere zeichnen sich durch eine hohe Hydrolysestabilität aus und eignen sich besonders als Vernetzermonomere.

Alternativ oder zusätzlich können die erfindungsgemäßen Dentalwerkstoffe neben den oben genannten Co-Monomeren ein oder mehrere säuregruppenhaltige radikalisch polymerisierbare Monomere (Haftmonomere) als Zusatzmonomere enthalten. Diese verleihen den Werkstoffen selbsthaftende und/oder selbstätzende Eigenschaften.

Die Verbindungen Formel I sind unter aciden Bedingungen stabil und lassen sich mit UV-Licht zur farbigen ringgeschlossenen Form schalten. Die Rückreaktion mit sichtbarem Licht läuft ebenfalls reversibel ab.

Bevorzugte säuregruppenhaltige Monomere sind polymerisationsfähige Carbonsäuren, Phosphonsäuren, Phosphorsäureester, und Sulfonsäuren.

Bevorzugte Carbonsäuren sind Maleinsäure, Acrylsäure, Methacrylsäure, 2-(Hydroxymethyl)acrylsäure, 4-(Meth)acryloyloxyethyltrimellitsäure, 10-Methacryloyloxydecylmalonsäure, N-(2-Hydroxy-3-methacryloyloxypropyl)-N-phenylglycin bzw. 4-Vinylbenzoesäure.

Bevorzugte Phosphonsäuremonomere sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentylphosphonsäure, 2-[4-(Dihydroxyphosphoryl)-2-oxa-butyl]-acrylsäure oder 2-[4-(Dihydroxyphosphoryl)-2-oxa-butyl]-acrylsäureethyl- und -2,4,6-trimethylphenylester.

Bevorzugte acide polymerisationsfähige Phosphorsäureester sind 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yl-dihydrogenphosphat.

Bevorzugte polymerisationsfähige Sulfonsäuren sind Vinylsulfonsäure, 4-Vinylphenylsulfonsäure oder 3-(Methacrylamido)-propylsulfonsäure.

Besonders bevorzugte Säuremonomere sind 4-(Meth)acryloyloxyethyltrimellitsäureanhydrid, 10-Methacryloyloxydecylmalonsäure, 2-Methacryloyloxyethylphosphonsäure, 2-Methacrylamidoethylphosphonsäure, 2-[4-(Dihydroxyphosphoryl)-2-oxa-butyl]-acrylsäure, 2-[4-(Dihydroxyphosphoryl)-2-oxa-butyl]-acrylsäureethyl- oder -2,4,6-trimethylphenylester, 2-Methacryloyloxypropylmono- oder -dihydrogenphosphat, 2-Methacryloyloxyethylmono- oder -dihydrogenphosphat, 2-Methacryloyloxyethylphenylhydrogenphosphat, 10-Methacryloyloxydecyl-dihydrogenphosphat, 6-(Methacrylamido)hexyldihydrogenphosphat und 1,3-Bis-(N-acryloyl-N-propyl-amino)-propan-2-yl-dihydrogenphosphat.

Die erfindungsgemäßen Dentalwerkstoffe enthalten mindestens einen Initiator für die radikalische Polymerisation.

Zur Initiierung der radikalischen Photopolymerisation sind Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil bevorzugt. Besonders bevorzugt werden Campherchinon und 2,2-Dimethoxy-2-phenyl-acetophenon und besonders bevorzugt α-Diketone in Kombination mit Aminen als Reduktionsmittel verwendet, wie z.B. 4-(Dimethylamino)benzoesäureethylester, N,N-Dimethylaminoethylmethacrylat, N,N-Dimethyl-sym.-xylidin oder Triethanolamin. Besonders geeignet sind auch Norrish-Typ-I-Photoinitiatoren, vor allem Acyl- oder Bisacylphosphinoxide, Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethylgermanium. Dabei lassen sich vorteilhaft auch Mischungen der verschiedenen Photoinitiatoren einsetzen, wie z.B. Dibenzoyldiethylgermanium in Kombination mit Campherchinon und 4-Dimethylaminobenzoesäureethylester.

Als Initiatoren für eine bei Raumtemperatur durchgeführte Polymerisation werden vorzugsweise Redox-Initiatorkombinationen, wie z.B. Kombinationen von Benzoylperoxid mit N,N-Dimethyl-sym.-xylidin oder N,N-Dimethyl-p-toluidin, verwendet. Darüber hinaus sind auch Redoxsysteme bestehend aus Peroxiden bzw. Hydroperoxiden und solchen Reduktionsmitteln, wie z.B. Ascorbinsäure, Barbiturate, Thioharnstoffe oder Sulfinsäuren, besonders geeignet.

Weiterhin enthalten die erfindungsgemäßen Dentalwerkstoffe vorzugsweise auch mindestens einen organischen oder besonders bevorzugt anorganischen partikulären Füllstoff. Bevorzugt sind Füllstoffe auf der Basis von Oxiden, wie SiO₂, ZrO₂ und TiO₂ bzw. Mischoxiden aus SiO₂, ZrO₂, ZnO und/oder TiO₂, nanopartikuläre oder mikrofeine Füllstoffe, wie pyrogene Kieselsäure oder Fällungskieselsäure (gewichtsmittlere Partikelgröße von 10-1000 nm) sowie Minifüllstoffe, wie Quarz-, Glaskeramik- oder röntgenopake Glaspulver von z.B. Barium- oder Strontiumaluminiumsilikatgläsern (gewichtsmittlere Partikelgröße von 0,2-10 µm). Weitere bevorzugte Füllstoffe sind röntgenopake Füllstoffe, wie Ytterbiumtrifluorid oder nanopartikuläres Tantal(V)-oxid bzw. Bariumsulfat oder Mischoxide von SiO₂ mit Ytterbium(III)-oxid oder Tantal(V)-oxid (gewichtsmittlere Partikelgröße von 10-1000 nm).

Zur Verbesserung des Verbundes zwischen den Füllstoffpartikeln und der vernetzten Polymerisationsmatrix können SiO₂-basierende Füllstoffe mit methacrylat-funktionalisierten Silanen, wie z.B. 3-Methacryloyloxypropyltrimethoxysilan, oberflächenmodifiziert werden. Zur Oberflächenmodifizierung von nichtsilikatischen Füllstoffen, z.B. von ZrO₂ oder TiO₂, können auch funktionalisierte saure Phosphate, wie z.B. 10-Methacryloyloxydihydrogenphospaphat eingesetzt werden.

Die erfindungsgemäßen Dentalwerkstoffe können in Abhängigkeit vom gewünschten Einsatzzweck vorzugsweise auch ein Lösungsmittel enthalten, insbesondere Wasser, Ethanol oder eine Mischung davon.

Gegebenenfalls können die erfindungsgemäß eingesetzten Zusammensetzungen außerdem weitere Additive enthalten, vor allem Stabilisatoren, wie z.B. Polymerisationsstabilisatoren, Aromastoffe, Farbmittel, mikrobiozide Wirkstoffe, fluoridionenabgebende Additive, optische Aufheller, Fluoreszensmittel, Weichmacher, und/oder UV-Absorber.

Dabei sind erfindungsgemäß solche Dentalmaterialien bevorzugt, die die folgenden Komponenten enthalten:
a) 0,0001 bis 5,0 Gew.-%, bevorzugt 0,001 bis 3,0 Gew.-% und besonders bevorzugt 0,01 bis 1,0 Gew.-% mindestens einer Verbindung der allgemeinen Formel I,
b) 0,01 bis 10 Gew.-%, besonders bevorzugt 0,1 bis 3,0 Gew.-% mindestens eines Initiators, und ggf.
c) 0 bis 80 Gew.-%, bevorzugt 0 bis 60 Gew.-% und besonders bevorzugt 5 bis 50 Gew.-% andere(s) Monomer(en), und ggf.
d) 0 bis 80 Gew.-% Füllstoff(e), und ggf.
e) 0 bis 70 Gew.-% Lösungsmittel.

Dentalwerkstoffe zur Verwendung als Zement oder Füllungskomposit weisen bevorzugt folgende Zusammensetzung auf:
a) 0,0001 bis 5,0 Gew.-%, bevorzugt 0,001 bis 3,0 Gew.-% und besonders bevorzugt 0,01 bis 1,0 Gew.-% mindestens einer Verbindung der allgemeinen Formel I,
b) 0,01 bis 10 Gew.-%, besonders bevorzugt 0,1 bis 3,0 Gew.-% mindestens eines Initiators,
c) 0 bis 50 Gew.-%, bevorzugt 0 bis 40 Gew.-% und besonders bevorzugt 5 bis 40 Gew.-% andere(s) Monomer(e),
d) 10 bis 80 Gew.-%, bevorzugt 20 bis 80 Gew.-%, besonders bevorzugt 30 bis 80 Gew.-% Füllstoff(e).

Dentalwerkstoffe zur Verwendung als Adhäsive oder Beschichtungsmaterial weisen bevorzugt folgende Zusammensetzung auf:
a) 0,0001 bis 5,0 Gew.-%, bevorzugt 0,001 bis 3,0 Gew.-% und besonders bevorzugt 0,01 bis 1,0 Gew.-% mindestens einer Verbindung der allgemeinen Formel I,
b) 0,01 bis 10 Gew.-%, besonders bevorzugt 0,1 bis 3,0 Gew.-% mindestens eines Initiators,
c) 0 bis 80 Gew.-%, bevorzugt 5 bis 60 Gew.-% und besonders bevorzugt 5 bis 50 Gew.-% andere(s) Monomer(e),
d) 0 bis 20 Gew.-% Füllstoff(e),
e) 0 bis 70 Gew.-%, bevorzugt 0 bis 60 Gew.-% und besonders bevorzugt 0 bis 50 Gew.-% Lösungsmitteln, insbesondere Wasser und/oder Ethanol.

Dentalwerkstoffe zur Herstellung von Prothesen oder künstlichen Zähnen weisen bevorzugt folgende Zusammensetzung auf:
a) 0,0001 bis 5,0 Gew.-%, bevorzugt 0,001 bis 3,0 Gew.-% und besonders bevorzugt 0,01 bis 1,0 Gew.-% mindestens einer Verbindung der allgemeinen Formel I,
b) 0,01 bis 10 Gew.-%, besonders bevorzugt 0,1 bis 3,0 Gew.-% mindestens eines Initiators, und
c) 0 bis 80 Gew.-%, bevorzugt 0 bis 60 Gew.-% und besonders bevorzugt 5 bis 50 Gew.-% andere(s) Monomer(e), und
d) 0 bis 40 Gew.-% Füllstoff(e).

Dentalwerkstoffe zur Herstellung von Inlays, Onlays, Kronen oder Brücken weisen bevorzugt folgende Zusammensetzung auf:
a) 0,0001 bis 5,0 Gew.-%, bevorzugt 0,001 bis 3,0 Gew.-% und besonders bevorzugt 0,01 bis 1,0 Gew.-% mindestens einer Verbindung der allgemeinen Formel I,
b) 0,01 bis 10 Gew.-%, besonders bevorzugt 0,1 bis 3,0 Gew.-% mindestens eines Initiators, und ggf.
c) 0 bis 60 Gew.-%, bevorzugt 0 bis 50 Gew.-% und besonders bevorzugt 5 bis 50 Gew.-% andere(s) Monomer(e), und ggf.
d) 10 bis 80 Gew.-%, bevorzugt 15 bis 80 Gew.-% und besonders bevorzugt 20 bis 80 Gew.-% Füllstoff(e).

Wenn nicht anders angegeben beziehen sich alle Mengenangaben auf die Gesamtmasse der Materialien. Die einzelnen Mengenbereiche können separat gewählt werden.

Besonders bevorzugt sind solche Werkstoffe, welche aus den genannten Komponenten bestehen. Weiterhin sind solche Werkstoffe bevorzugt, bei denen die einzelnen Komponenten jeweils aus den oben genannten bevorzugten und besonders bevorzugten Stoffen ausgewählt sind.

Die erfindungsgemäßen Werkstoffe eignen sich besonders als Dentalwerkstoffe, insbesondere als dentale Zemente, Füllungskomposite und Verblendmaterialien sowie als Materialien zur Herstellung von Prothesen, künstlichen Zähnen, Inlays, Onlays, Kronen und Brücken. Sie zeichnen sich durch reversible photochrome Eigenschaften aus.

Die Dentalwerkstoffe eignen sich primär zur intraoralen Anwendung durch den Zahnarzt zur Restauration geschädigter Zähne (klinische Materialien), d.h. zur therapeutischen Anwendung, z.B. als dentale Zemente, Füllungskomposite und Verblendmaterialien. Sie können aber auch extraoral eingesetzt werden, beispielsweise bei der Herstellung oder Reparatur von Dentalrestaurationen, wie Prothesen, künstlichen Zähnen, Inlays, Onlays, Kronen und Brücken (technische Materialien).

Die Erfindung wird im Folgenden anhand eines Ausführungsbeispiels näher erläutert.

### Ausführungsbeispiel

### Beispiel 1:

### Synthese der polymerisationsfähigen photochromen Verbindung 3,3,4,4,5,5-Hexafluor-1,2-bis(5-methacryloyloxymethyl-2-methyl-3-thienyl)cyclopent-1-en

### 1. Stufe: 4-Brom-5-methylthiophen-2-carboxaldehyd

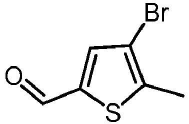

Zu einer Lösung von 5-Methyl-2-thiophencarboxaldehyd (12,62 g; 97,9 mmol) in Essigsäure (80 ml) wurde unter Lichtausschluss eine Lösung von Brom (19,18 g; 0,12 mol) in Essigsäure (50 ml) zugetropft, wobei die Temperatur durch Kühlung im Wasserbad unter 30 °C gehalten wurde. Das Reaktionsgemisch wurde 72 h bei RT gerührt und dann vorsichtig in gesättigte wässrige Na₂CO₃-Lösung (500 ml) gegossen. Nach Abklingen der Gasentwicklung wurde Diethylether (400 ml) zugegeben und die Phasen wurden getrennt. Die Wasserphase wurde mit Diethylether (2x 100 ml) extrahiert. Die vereinigten organischen Phasen wurden mit gesättigter wässriger NaHCO₃-Lösung (150 ml) gewaschen, über Na₂SO₄ getrocknet, filtriert und am Rotationsverdampfer eingeengt. Das Rohprodukt wurde mittels Säulenchromatographie gereinigt (SiO₂, n-Hexan/Ethylacetat 9:1; R*_{f}* = 0,47). Man erhielt 14,67 g (71,5 mmol; 73%) eines gelblichen Feststoffs.
¹H-NMR (CDCl₃, 400 MHz): δ = 2.48 (s, 3H; CH₃), 7.59 (s, 1H, =CH), 9.77 (s, 1H, CHO).
¹³C-NMR (CDCl₃, 100.6 MHz): δ = 15.9 (CH₃), 111.2 (=C), 138.7 (=CH), 140.1 (=C), 145.8 (=C), 181.6 (C=O).

### 2. Stufe: 4-Brom-2-hydroxymethyl-5-methylthiophen

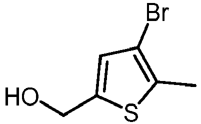

Eine Lösung von 4-Brom-5-methylthiophen-2-carboxaldehyd (24,20 g; (0,118 mol) in Ethanol (250 ml) wurde bei 0 °C mit Natriumborhydrid (5,36 g; 0,142 mol) versetzt. Das Reaktionsgemisch wurde 1 h bei 0 °C und 2 h bei Umgebungstemperatur gerührt. Dann wurden gesättigte wässrige NH₄Cl-Lösung (100 ml), Wasser (200 ml) und Ethylacetat (300 ml) zugegeben und die Phasen wurden getrennt. Die Wasserphase wurde mit Ethylacetat (100 ml) extrahiert. Die vereinigten organischen Phasen wurden mit gesättigter wässriger NaHCO₃-Lösung (100 ml) und gesättigter wässriger NaCl-Lösung (100 ml) gewaschen, über Na₂SO₄ getrocknet, filtriert und am Rotationsverdampfer eingeengt. Das Rohprodukt wurde mittels Säulenchromatographie gereinigt (SiO₂, *n-*Hexan/Ethylacetat 4:1; R*_{f}* = 0,35). Man erhielt 17,72 g (73%) einer gelblichen Flüssigkeit.
¹H-NMR (CDCl₃, 400 MHz): δ = 2.35 (s, 3H; CH₃), 3.15 (t, 1H; *J* = 4.4 Hz; OH), 4.60 (d, 2H; *J* = 4.4 Hz; CH₂), 6.74 (s, 1H; =CH) .
¹³C-NMR (CDCl₃, 100.6 MHz): δ = 14.7 (CH₃), 59.4 (CH₂OH), 108.2 (=C), 127.7 (=CH), 134.2 (=C), 140.8 (=C).

### 3. Stufe: (4-Bromo-5-methyl-thiophen-2-ylmethoxy)-tert.-butyl-dimethylsilan

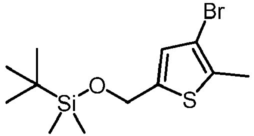

Imidazol (6,36 g; 93,5 mmol) wurde zu einer Lösung von 4-Brom-2-hydroxymethyl-5-methylthiophen (17,60 g; 85,0 mmol) und tert.-Butyldimethylchlorsilan (14,09 g; 93,5 mmol) in wasserfreiem Dichlormethan (100 ml) zugegeben und die Suspension wurde bei RT gerührt. Nach 2 h wurde das Reaktionsgemisch mit verdünnter Salzsäure (1*N*; 100 ml) gewaschen, über Na₂SO₄ getrocknet, filtriert und am Rotationsverdampfer eingeengt. Das Rohprodukt wurde in n-Hexan (50 ml) gelöst und über Kieselgel filtriert (SiO₂, *n*-Hexan). Das Filtrat wurde am Rotationsverdampfer eingeengt und der Rückstand am Feinvakuum getrocknet. Man erhielt 25,84 g (95%) eines farblosen Öls.
¹H-NMR (CDCl₃, 400 MHz): δ = 0.11 (s, 6H; Si-CH₃), 0.93 (s, 9H; C-CH₃), 2.36 (s, 3H; CH₃), 4.75 (s, 2H; O-CH₂), 6.70 (s, 1H; =CH) .
¹³C-NMR (CDCl₃, 100.6 MHz): δ = -5.3 (CH₃), 14.7 (CH₃), 18.3 (C), 25.8 (CH₃), 60.5 (CH₂), 107.9 (=C), 126.0 (=CH), 133.1 (=C), 142.2 (=C).
²⁹Si-NMR (CDCl₃, 79.5 MHz): δ = 21.6.

### 4. Stufe: 3,3,4,4,5,5-Hexafluor-1,2-bis[(tert.-butyldimethylsilyl)oxymethyl-2-methyl-3-thienyl)cyclopent-1-en

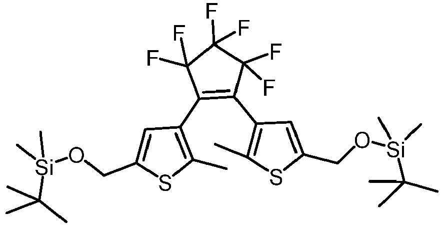

Eine Lösung von (4-Bromo-5-methyl-thiophen-2-ylmethoxy)-tert.-butyl-dimethylsilan (25,74 g; 80,0 mmol) in wasserfreiem Tetrahydrofuran (100 ml) unter Argon wurde auf -75 °C abgekühlt. *n*-Butyllithium (2,5M in n-Hexan; 32,6 ml; 82,0 mmol) wurde zugetropft und die gelbe Lösung wurde 2 h bei -75 °C gerührt. Octafluorcyclopenten (8,48 g; 40,0 mmol) wurde zugegeben und das Reaktionsgemisch wurde über Nacht im auftauenden Kältebad gerührt. Nach 20 h wurde das Reaktionsgemisch mit Wasser (100 ml) und Ethylacetat (300 ml) versetzt und die Phasen wurden getrennt. Die organische Phase wurde mit Wasser (2x 100 ml) gewaschen. Die vereinigten Wasserphasen wurden mit Ethylacetat (100 ml) reextrahiert. Die vereinigten organischen Phasen wurden mit gesättigter wässriger NaCl-Lösung (100 ml) gewaschen, über Na₂SO₄ getrocknet, filtriert und am Rotationsverdampfer eingeengt. Das Rohprodukt wurde mittels Säulenchromatographie gereinigt (SiO₂, n-Hexan/Ethylacetat 20:1; R*_{f}* = 0,63). Man erhielt 19,46 g (74%) einer bräunlichen Flüssigkeit.
¹H-NMR (CDCl₃, 400 MHz): δ = 0.11 (s, 12H; Si-CH₃), 0.94 (s, 18H; C-CH₃), 1.88 (s, 6H; CH₃), 4.80 (s, 4H; O-CH₂), 6.87 (s, 2H; =CH).
¹³C-NMR (CDCl₃, 100.6 MHz): δ = -5.3 (CH₃), 14.4 (CH₃), 18.3 (C), 25.8 (CH₃), 60.6 (CH₂), 111.1 (m; CF₂), 113.7 (m; CF₂), 116.2 (m; CF₂), 118.8 (m; CF₂), 123.1 (=CH), 124.3 (=C), 135.8 (m, =C-CF₂), 141.1 (=C), 143.5 (=C).
¹⁹F-NMR (CDCl₃, 376.5 MHz): δ = -131.9 (2F), -110.0 (4F) . ²⁹Si-NMR (CDCl₃, 79.5 MHz): δ = 21.7.

### 5. Stufe: 3,3,4,4,5,5-Hexafluor-1,2-bis(5-hydroxymethyl-2-methyl-3-thienyl)cyclopent-1-en

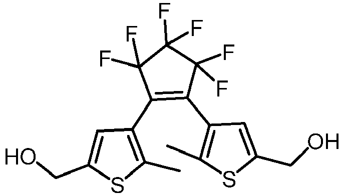

Zu einer Lösung von 3,3,4,4,5,5-Hexafluor-1,2-bis[(*tert*.-butyldimethylsilyl)oxymethyl-2-methyl-3-thienyl)cyclopent-1-en (19,20 g; 29,2 mmol) in Tetrahydrofuran (100 ml) wurde Tetrabutylammoniumfluorid (75% in Wasser; 25,00 g; 66,9 mmol) zugetropft. Die Reaktionslösung wurde 4 h RT gerührt, dann mit gesättigter wässriger NH₄Cl-Lösung (100 ml) und Ethylacetat (100 ml) versetzt und die Phasen wurden getrennt. Die organische Phase wurde mit Wasser (2x 100 ml) und gesättigter wässriger NaCl-Lösung (100 ml) gewaschen, über Na₂SO₄ getrocknet, filtriert und am Rotationsverdampfer eingeengt. Das Rohprodukt wurde mittels Säulenchromatographie gereinigt (SiO₂, *n-*Hexan/Ethylacetat 1:1; R*_{f}* = 0,38). Man erhielt 4,35 g (10.2 mmol; 35%) eines farblosen Feststoffs.
¹H-NMR (CDCl₃, 400 MHz): δ = 1.87 (s, 6H; CH₃), 4.26 (s, 2H; OH), 4.68 (s, 4H; O-CH₂), 6.91 (s, 2H; =CH) .
¹³C-NMR (CDCl₃, 100.6 MHz): δ = 14.3 (CH₃), 59.1 (CH₂), 110.8 (m; CF₂), 113.4 (m; CF₂), 116.0 (m; CF₂), 118.5 (m; CF₂), 124.0 (=CH), 135.7 (m, =C-CF₂), 141.4 (=C), 143.1 (=C) .
¹⁹F-NMR (CDCl₃, 376.5 MHz): δ = -131.9 (2F), -110.0 (4F) .

### 6. Stufe: 3,3,4,4,5,5-Hexafluor-1,2-bis(5-methacryloyloxymethyl-2-methyl-3-thienyl)cyclopent-1-en

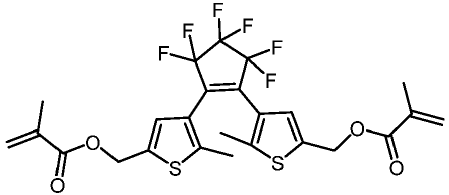

Zu einer Lösung von 3,3,4,4,5,5-Hexafluor-1,2-bis(5-hydroxymethyl-2-methyl-3-thienyl)cyclopent-1-en (4,25 g; 9,9 mmol), Triethylamin (2,21 g; 21,8 mmol) und N,N-Dimethylaminopyridin (0,24 g; 2,0 mmol) in wasserfreiem Dichlormethan (80 ml) wurde bei -5 °C eine Lösung von Methacrylsäureanhydrid (3,21 g; 20,8 mmol) in Dichlormethan (20 ml) zugetropft. Das Reaktionsgemisch wurde 3 h bei -5 °C und anschließend bei Umgebungstemperatur gerührt. Nach 20 h wurde die Lösung mit Wasser (3x 100 ml) und gesättigter wässriger NaCl-Lösung (100 ml) gewaschen, über Na₂SO₄ getrocknet, filtriert und am Rotationsverdampfer eingeengt. Das Rohprodukt wurde mittels Säulenchromatographie gereinigt (SiO₂, n-Hexan/Ethylacetat 9:1; R*_{f}* = 0,40). Man erhielt 4,68 g (8,3 mmol; 84%) eines farblosen Feststoffs (Smp.: 58 °C).
¹H-NMR (CDCl₃, 400 MHz): δ = 1.86 (s, 6H; CH₃), 1.95 (m, 6H; CH₃), 5.24 (s, 4H; O-CH₂), 5.61 (m, 2H; =CH), 6.14 (m, 2H; =CH); 7.05 (s, 2H; =CH).
¹³C-NMR (CDCl₃, 100.6 MHz): δ = 14.2 (CH₃), 18.2 (CH₃), 60.5 (CH₂), 110.9 (m; CF₂), 113.4 (m; CF₂), 116.0 (m; CF₂), 118.5 (m; CF₂), 124.3 (=C), 126.3 (=CH₂), 127.5 (=CH), 135.8 (=C), 136.1 (m, =C-CF₂), 136.4 (=C), 143.2 (=C), 166. 9 (C=O).
¹⁹F-NMR (CDCl₃, 376.5 MHz): δ = -131.9 (2F), -110.2 (4F).
   IR (neat): 2958 (w), 1716 (s), 1637 (m), 1561 (w), 1441 (m), 1404 (w), 1380 (w), 1337 (m), 1316 (m), 1273 (s), 1191 (m), 1136 (vs), 1109 (vs), 1046 (s), 1011 (m), 984 (vs), 942 (s), 898 (m), 856 (m), 813 (s), 740 (m), 709 (w), 656 (m), 635 (w) cm⁻¹.
Analyse berechnet für C₂₅H₂₂F₆O₄S₂: C, 53.19; H, 3.93; S, 11.36. Gefunden: C, 53.20; H, 3.76; S, 11.10.

## Patentansprüche

1. Radikalisch polymerisierbarer Dentalwerkstoff, **dadurch gekennzeichnet, dass** er mindestens eine photochrome Verbindung der Formel (I), 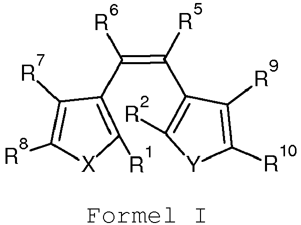 in der die Variablen die folgenden Bedeutungen haben:
X,Y jeweils S;
R¹,R² jeweils Methyl;
R⁵;R⁶ bilden zusammen eine -(CH₂)ₙ- Gruppe, wobei n 3 ist und wobei in der -(CH₂)- Gruppe ein oder mehrere H-Atome durch F ersetzt sein können;
R⁷,R⁹ unabhängig voneinander jeweils H oder ein C₁-C₃-Alkylrest;
R⁸,R¹⁰ unabhängig voneinander jeweils ein C₁-C₆-Alkylrest, der durch O oder -O-C(=O)-NH- unterbrochen sein kann und der eine endständige radikalisch polymerisierbare Gruppe trägt,
mindestens ein radikalisch polymerisierbares Monomer und mindestens einen Initiator für die radikalische Polymerisation enthält.

2. Dentalwerkstoff nach Anspruch 1, in dem alle H-Atome in R⁵ und R⁶ durch F ersetzt sind.

3. Dentalwerkstoff nach Anspruch 1 oder 2, bei dem die radikalisch polymerisierbare Gruppe in R⁸ und R¹⁰ eine Methacrylatgruppe ist.

4. Dentalwerkstoff nach einem der vorangehenden Ansprüche, in dem das radikalisch polymerisierbare Monomer ein mono- oder polyfunktionelles Methacrylsäurederivat ist.

5. Dentalwerkstoff nach einem der vorangehenden Ansprüche, in dem der Initiator für die radikalische Polymerisation ein Photoinitiator ist.

6. Dentalwerkstoff nach einem der Ansprüche 1 bis 5, der zusätzlich mindestens einen partikulären Füllstoff enthält.

7. Dentalwerkstoff nach einem der Ansprüche 1 bis 6, der
a) 0,0001 bis 5,0 Gew.-%, bevorzugt 0,001 bis 3,0 Gew.-% und besonders bevorzugt 0,01 bis 1,0 Gew.-% mindestens einer Verbindung der allgemeinen Formel I,
b) 0,01 bis 10 Gew.-%, besonders bevorzugt 0,1 bis 3,0 Gew.-% mindestens eines Initiators, und ggf.
c) 0 bis 80 Gew.-%, bevorzugt 0 bis 60 Gew.-% und besonders bevorzugt 5 bis 50 Gew.-% andere(s) Monomer(e), und ggf.
d) 0 bis 80 Gew.-% Füllstoff(e) und ggf.
e) 0 bis 70 Gew.-% Lösungsmittel,
enthält, jeweils bezogen auf die Gesamtmasse des Dentalwerkstoffs.

8. Werkstoff nach Anspruch 7 zur Verwendung als dentaler Zement oder dentales Füllungskomposit, der
a) 0,0001 bis 5,0 Gew.-%, bevorzugt 0,001 bis 3,0 Gew.-% und besonders bevorzugt 0,01 bis 1,0 Gew.-% mindestens einer Verbindung der allgemeinen Formel I,
b) 0,01 bis 10 Gew.-%, besonders bevorzugt 0,1 bis 3,0 Gew.-% mindestens eines Initiators,
c) 0 bis 50 Gew.-%, bevorzugt 0 bis 40 Gew.-% und besonders bevorzugt 5 bis 40 Gew.-% andere(s) Monomer(e),
d) 10 bis 80 Gew.-%, bevorzugt 20 bis 80 Gew.-%, besonders bevorzugt 30 bis 80 Gew.-% Füllstoff(e) enthält.

9. Werkstoff nach Anspruch 7 zur Verwendung als dentales Adhäsiv oder Beschichtungsmaterial, der
a) 0,0001 bis 5,0 Gew.-%, bevorzugt 0,001 bis 3,0 Gew.-% und besonders bevorzugt 0,01 bis 1,0 Gew.-% mindestens einer Verbindung der allgemeinen Formel I,
b) 0,01 bis 10 Gew.-%, besonders bevorzugt 0,1 bis 3,0 Gew.-% mindestens eines Initiators,
c) 0 bis 80 Gew.-%, bevorzugt 5 bis 60 Gew.-% und besonders bevorzugt 5 bis 50 Gew.-% andere(s) Monomer(e),
d) 0 bis 20 Gew.-% Füllstoff(e),
e) 0 bis 70 Gew.-%, bevorzugt 0 bis 60 Gew.-% und besonders bevorzugt 0 bis 50 Gew.-% Lösungsmittel, insbesondere Wasser und/oder Ethanol enthält.

10. Werkstoff nach einem der Ansprüche 1 bis 9 zur intraoralen Verwendung zur Restauration geschädigter Zähne.

11. Werkstoff nach Anspruch 10 zur therapeutischen Verwendung als dentaler Zement, dentales Füllungskomposit, dentales Adhäsiv oder Verblendmaterial.

12. Verwendung einer Verbindung der Formel I, wie in Anspruch 1 definiert, zur Herstellung eines photochromen Dentalwerkstoffs.

## Claims

1. Radically polymerisable dental material, **characterised in that** it comprises at least one photochromic compound of Formula (I), 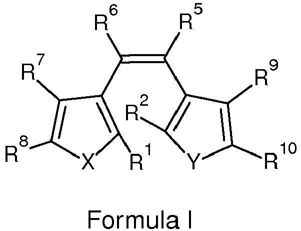 in which the variables have the following meanings:
X, Y are each S;
R¹, R² are each methyl;
R⁵; R⁶ together form a -(CH₂)ₙ- group, wherein n is 3 and wherein one or more H atoms may be replaced in the -(CH₂)ₙ- group by F;
R⁷, R⁹ independently of each other are each H or a C₁-C₃ alkyl group;
R⁸, R¹⁰ independently of each other are each a C₁-C₆ alkyl group that may be interrupted by O or -O-C(=O)-NH-, and which may carry a terminal radically polymerisable group,
at least one radically polymerisable monomer and at least one initiator for radical polymerisation.

2. Dental material according to claim 1, in which all H atoms in R⁵ and R⁶ are replaced by F.

3. Dental material according to claim 1 or 2, in which the radically polymerisable group in R⁸ and R¹⁰ is a methacrylate group.

4. Dental material according to one of the preceding claims, in which the radically polymerisable monomer is a mono- or polyfunctional methacrylic acid derivative.

5. Dental material according to one of the preceding claims, in which the initiator for the radical polymerisation is a photoinitiator.

6. Dental material according to one of claims 1 to 5, which additionally comprises at least one particulate filler.

7. Dental material according to one of claims 1 to 6, which comprises
a) 0.0001 to 5.0 wt %, preferably 0.001 to 3.0 wt % and particularly preferably 0.01 to 1.0 wt % of at least one compound of the general formula I,
b) 0.01 to 10 wt %, particularly preferably 0.1 to 3.0 wt % of at least one initiator, and optionally
c) 0 to 80 wt %, preferably 0 to 60 wt % and particularly preferably 5 to 50 wt % of other monomer(s), and optionally
d) 0 to 80 wt % filler(s), and optionally
e) 0 to 70 wt % solvent,
in each case relative to the total mass of the dental material.

8. Material according to claim 7 for use as dental cement or dental filling composite, which comprises
a) 0.0001 to 5.0 wt %, preferably 0.001 to 3.0 wt % and particularly preferably 0.01 to 1.0 wt % of at least one compound of the general formula I,
b) 0.01 to 10 wt %, particularly preferably 0.1 to 3.0 wt % of at least one initiator,
c) 0 to 50 wt %, preferably 0 to 40 wt % and particularly preferably 5 to 40 wt % of other monomer(s),
d) 10 to 80 wt %, preferably 20 to 80 wt %, particularly preferably 30 to 80 wt % filler(s).

9. Material according to claim 7 for use as dental cement or dental filling composite, which comprises
a) 0.0001 to 5.0 wt %, preferably 0.001 to 3.0 wt % and particularly preferably 0.01 to 1.0 wt % of at least one compound of the general formula I,
b) 0.01 to 10 wt %, particularly preferably 0.1 to 3.0 wt % of at least one initiator,
c) 0 to 80 wt %, preferably 5 to 60 wt % and particularly preferably 5 to 50 wt % of other monomer(s),
d) 0 to 20 wt % of filler(s)
e) 0 to 70 wt %, preferably 0 to 60 wt % and particularly preferably 0 to 50 wt % solvent, in particular water and/or ethanol.

10. Material according to one of claims 1 to 9 for intraoral use to restore damaged teeth.

11. Material according to claim 10 for therapeutic use as a dental cement, dental filling composite, dental adhesive or veneering material.

12. Use of a compound of Formula I, as defined in claim 1, for the preparation of a photochromic dental material.

## Revendications

1. Matériau dentaire polymérisable par voie radicalaire, **caractérisé en ce qu'**il contient au moins un composé photochrome de formule (I), 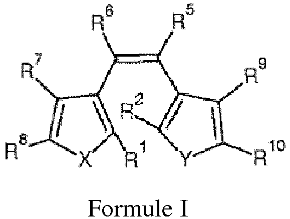 dans laquelle les variables ont les significations suivantes :
X, Y représentent chacun un atome de soufre ;
R¹, R² représentent chacun un groupe méthyle ;
R⁵, R⁶ forment ensemble un groupe -(CH₂)ₙ-, n étant égal à 3 et un ou plusieurs atome(s) d'hydrogène dans le groupe -(CH₂)- pouvant être remplacé(s) par un(des) atome(s) de fluor ;
R⁷, R⁹ représentent chacun, indépendamment l'un de l'autre, un atome d'hydrogène ou un radical alkyle en C₁-C₃ ;
R⁸, R¹⁰ représentent chacun, indépendamment l'un de l'autre, un radical alkyle en C₁-C₆, qui peut être interrompu par un atome d'oxygène ou par un groupe -O-C(=O)-NH- et qui porte un groupe apte à la polymérisation radicalaire en bout de chaîne,
au moins un monomère polymérisable par voie radicalaire et au moins un amorceur pour la polymérisation radicalaire.

2. Matériau dentaire selon la revendication 1, dans lequel tous les atomes d'hydrogène dans R⁵ et R⁶ sont remplacés par des atomes de fluor.

3. Matériau dentaire selon la revendication 1 ou 2, dans lequel le groupe apte à la polymérisation radicalaire dans R⁸ et R¹⁰ est un groupe méthacrylate.

4. Matériau dentaire selon l'une quelconque des revendications précédentes, dans lequel le monomère polymérisable par voie radicalaire est un dérivé d'acide méthacrylique mono- ou polyfonctionnel.

5. Matériau dentaire selon l'une quelconque des revendications précédentes, dans lequel l'amorceur pour la polymérisation radicalaire est un photoamorceur.

6. Matériau dentaire selon l'une quelconque des revendications 1 à 5, qui contient additionnellement au moins une charge particulaire.

7. Matériau dentaire selon l'une quelconque des revendications 1 à 6, qui contient
a) 0,0001 à 5,0 % en poids, de préférence 0,001 à 3,0 % en poids et de façon particulièrement préférée 0,01 à 1,0 % en poids d'au moins un composé de formule générale I,
b) 0,01 à 10 % en poids, de façon particulièrement préférée 0,1 à 3,0 % en poids d'au moins un amorceur, et éventuellement
c) 0 à 80 % en poids, de préférence 0 à 60 % en poids et de façon particulièrement préférée 5 à 50 % en poids d'un autre/d'autres monomère(s), et éventuellement
d) 0 à 80 % en poids de charge(s) et éventuellement
e) 0 à 70 % en poids de solvant,
chaque fois par rapport à la masse totale du matériau dentaire.

8. Matériau selon la revendication 7, destiné à l'utilisation en tant que ciment dentaire ou composite d'obturation dentaire, qui contient
a) 0,0001 à 5,0 % en poids, de préférence 0,001 à 3,0 % en poids et de façon particulièrement préférée 0,01 à 1,0 % en poids d'au moins un composé de formule générale I,
b) 0,01 à 10 % en poids , de façon particulièrement préférée 0,1 à 3,0 % en poids d'au moins un amorceur,
c) 0 à 50 % en poids, de préférence 0 à 40 % en poids et de façon particulièrement préférée 5 à 40 % en poids d'un autre/d'autres monomère(s),
d) 10 à 80 % en poids, de préférence 20 à 80 % en poids, de façon particulièrement préférée 30 à 80 % en poids de charge(s).

9. Matériau selon la revendication 7, destiné à l'utilisation en tant qu'adhésif dentaire ou en tant que matériau de revêtement, qui contient
a) 0,0001 à 5,0 % en poids, de préférence 0,001 à 3,0 % en poids et de façon particulièrement préférée 0,01 à 1,0 % en poids d'au moins un composé de formule générale I,
b) 0,01 à 10 % en poids , de façon particulièrement préférée 0,1 à 3,0 % en poids d'au moins un amorceur,
c) 0 à 80 % en poids, de préférence 5 à 60 % en poids et de façon particulièrement préférée 5 à 50 % en poids d'un autre/d'autres monomère(s),
d) 0 à 20 % en poids de charge(s),
e) 0 à 70 % en poids, de préférence 0 à 60 % en poids et de façon particulièrement préférée 0 à 50 % en poids de solvant, en particulier d'eau et/ou d'éthanol.

10. Matériau selon l'une quelconque des revendications 1 à 9, destiné à l'utilisation intra-orale pour la restauration de dents abîmées.

11. Matériau selon la revendication 10, destiné à l'utilisation thérapeutique en tant que ciment dentaire, composite d'obturation dentaire, adhésif dentaire ou matériau de placage.

12. Utilisation d'un composé de formule I, tel que défini dans la revendication 1, pour la préparation d'un matériau dentaire photochrome.
